Europäisches Patentamt

European Patent Office

Office européen des brevets

(11) Publication number: **0 563 428 A1**

## EUROPEAN PATENT APPLICATION

(21) Application number: **92105732.9**

(22) Date of filing: **02.04.92**

(51) Int. Cl.⁵: **A61N 5/02**

(43) Date of publication of application:
**06.10.93 Bulletin 93/40**

(84) Designated Contracting States:
**AT BE CH DE DK ES FR GB GR IT LI NL PT SE**

(71) Applicant: **Hauzer, Franciscus Johannes Matheus**
**Koelderstraat 21**
**NL-5916 NH Venlo(NL)**

(72) Inventor: **Borisovith, Golant Mikhail**
**Str. Voksalnay 17-137**
**141120 Fryazino, Moscow Region(SU)**
Inventor: **Welgelmovith, Poslavsky Mikhail**
**Kashirskoe Way 82-152**

**115569 Moscow(SU)**
Inventor: **Pavlovna, Bazhanova Taisia**
**Str. Lenina 27-33**
**114420 Fryazino, Moscow Region(SU)**
Inventor: **Borisovna, Rebrova Tatyana**
**Str. Institutskaya 12-4**
**141120 Fryazino, Moscow Region(SU)**

(74) Representative: **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Postfach 22 16 11**
**D-80506 München (DE)**

(54) **Yav-1 EHF therapy apparatus.**

(57) A method for EHF-therapy consists in that an irradiation signal is shaped as an alternation of an effective signal of nonthermal-intensity EHF-radiation and a spacing that follows the effective signal, and the duration of each effective signal is shorter than the duration of the spacing that follows the effective signal. A device for carrying the method into effect incorporates a generator of nonthermal-intensity EHF-radiation, a signal chopper and a radiator, both being connected to the EHF-radiation generator through the portions of an EHF-channel, and a means for shaping an irradiation signal appearing as an alternation of an effective signal of EHF-radiation and a spacing that follows the effective signal, which means is connected to the signal chopper.

FIG. 1

EP 0 563 428 A1

Field of the Invention

The invention relates to therapeutic means and is concerned more particularly with methods and devices for EHF-(millimetric-wave) therapy.

The invention is applicable for acceleration of the treatment process in internal medicine, surgery and for treatment of advanced diseases and severe affections of human organism, especially in elderly-age patients.

Background of the Invention

Known in the present state of the art are EHF-therapy methods based on continuous exposure of the patient's organism to the effect of EHF-signals of low nonthermal intensity causing the heating of the tissues involved by not more than $0.1\,^{\circ}C$ at resonance frequencies, applied within a period of from 0.5 to 1 hour, a total treatment course consisting as a rule of several sessions. (cf. the journal 'Sovetskaya meditsina' (Soviet medicine) No. 1, 1989, Moscow, 'Treatment of peptic ulcers with EHF electromagnetic radiation", pp.29-31 by M.Y. Poslavsky, A.S. Parfionov, I.M.Korochkin, O.F. Zdavovich, D.O.Abshilava (in Russian).

A curative effect produced by EHF radiation is underlain by simulation of signals generated by organism's cells in cases of disturbed functioning thereof, said signals afterwards controlling over diverse energy-restoring processes.

Signals used in EHF-therapy are generated by EHF-therapy units.

Prior-art EHF-therapy units known and used nowadays differ from one another in the range of working frequencies, that is, the working frequencies fall within the range recommended for an EHF-channel having a cross-sectional area of $2.6 \bullet 5.2$ mm$^2$ or lie in the range recommended for use in an EHF-channel with a cross-sectional area of $1.8 \bullet 3.6$ mm$^2$ (cf. the journal "Elektronnaya promyshlennost" (Electronic industry) No. 1, 1985, Moscow, "Apparatus for local curative irradiation with EHF electromagnetic waves of nonthermal intensity", p.52 by M.B.Golant, Ju.V.Dedik, N.A.Krugliakov, T.B.Rebrova, A.S.Fyodorov, S.G.Yanchenko (in Russian).

The heretofore-known units such as the aforediscussed ones operating in a continuous mode, compirse a power source, a generator of EHF wave an EHF-channel with a ferrite isolator, a radiator, and an indication circuit incorporating a coupler, a high-Q resonator, and a detector head.

Nonthermal-intensity EHF-radiation is applied to the radiator connected to the generator through the EHF-channel, whereupon said radiation is applied to the patient's organism, thereby affording a curative effect without resorting to any drugs, costly and scarcely available ones inclusive, cutting down the treatment time, rendering the curative process more effective, reducing untoward side effects, and adding to the immune status.

However, efficacy of said method and device is reduced with an increased degree of severity and inveteracy of diseases, as well as in cases where they are applied for treatment of elderly-age patients. For instance, a positive dynamics of the restorative process in the initial period of treatment may afterwards be followed by retardation of that process or even by its stopping before the affection is corrected and the patient recovers.

Summary of the Invention

It is an object of the invention to provide a method and a device for EHF-therapy, that would make it possible to increase the efficacy of patients' treatment.

It is another object of the invention to cut down the treatment period.

It is one more object of the invention to carry out treatment of severe and inveterate diseases.

It is still more object of the invention to perform treatment of elderly-age patients.

The foregoing and further objects are accomplished due to the fact that there is selected nonthermal-intensity EHF-radiation and a patient's organism is exposed to the effect of said radiation to obtain a curative effect, according to the invention, an irradiation signal is shaped as a several alternating consecutive cycles, each consisting of an effective EHF-radiation signal followed by a spacing, and the duration of each effective signal is less than the duration of the spacing the follows said effective signal.

It is expedient that the duration of an effective signal be selected within 30 to 1200 s.

Said objects are accomplished also due to the fact a device for EHF-therapy of human diseases, comprising a nonthermal-intensity EHF-radiation generator and a radiator interconnected with said EHF-generator through an EHF-channel, according to the invention, incorporates a radiation signal shaping circuit, said radiation signal appearing as an effective signal followed by a spacing, wherein duration of each effective signal is less than the duration of the spacing, and a signal chopper connected to the radiation signal shaping circuit and interposed between the EHF-radiation generator and the radiator.

Brief Description of the Drawings

Further objects and advantages of the present invention will become evident hereinbelow from a consideration of a detailed description of some specific exemplary embodiments thereof with reference to the accompanying drawings, wherein:

FIG. 1 is a graphic representation of a time spent for attaining an effect of complete synchronisation as the duration of the effective signal;

FIG. 2 is a block diagram of a device for EHF-therapy, according to the invention; and

FIG. 3 is a block diagram of a means for shaping a radiation signal, according to the invention.

Detailed Description of the Invention

The herein-proposed method for EHF-therapy of human organism is concerned with the following experimentally trialled and theoretically substantiated reasons. Resonance bands, wherein a positive organism's response to EHF-radiation takes place, i.e., such a response that manifests itself in intensification of the reparative process, are very narrow and ranges within $10^{-3}$ and $10^{-4}$ of the working frequency bandwidth.

The presence of individual features of the same diseases in every patient results in that in some specific cases resonance frequencies may disagree. This in turn impedes badly the tuning of the devices to a frequency optimal for every particular patient. That is why an actual effect of EHF-therapy is produced at frequencies differing from those determined by the disease, that is, the oscillation frequencies that control over the reparative process in an intact healthy organism. The aforesaid frequencies are generated with the aid of transient protein substructures forming on cell membranes in response to disturbed functioning of cells. The aforesaid substructures compise the protein molecules at least one of the resonance frequency of which agrees with the frequency of oscillations excited in the cell membranes as a result of the upset normal functioning thereof. Frequency coincidence contributes to pulling of just these protein molecules towards the membrane by virtue of the EHF-field of the waves propagating over said membrane. However, when the organism is exposed to the effect of external electromagnetic waves at frequencies differing from those determined by the disease, molecules having resonance frequencies equal to oscillation frequencies acting on the organism from outside start to be pulled towards the protein substructures.

If such an effect had been followed by establishing oscillations at the frequencies differing from those determined by the disease they should not contribute to organism reparation and its recovery.

However, as it have been demonstrated by theoretical and experimental studies, so long as the number of the membrane-attracted protein molecules having resonance frequencies other than those determined by the disease causative of cell dysfunctioning, is substantially smaller than the number of the membrane-adhered protein molecules having resonance frequencies determined by the disease, the cells generate oscillations at the frequencies determined by the disease, the generation process occurring in the time intervals between the external effects. The presence of a number of protein molecules having other resonance frequencies in this case even makes for acceleration of the reparative process, that is, the elements of the protein substructures gain in size and penetrate more deeply into the cytoplasm, which expedites pulling of fresh protein molecules having a required resonance frequency, towards the membrane. As a result, generation is intensified, thus accelerating the reparative process.

However, when the external radiation is too intensive so that a lower amount of protein molecules is pulled towards the membrane during the spacing intervals than in the course of an EHF-therapy session, the number of protein molecules in the substructures having the frequencies differing from those determined by the disease will exceed in said substructures the number of molecules having resonance frequencies determined by the disease. In such cases oscillations generated by the cells in the time spacings between the therapeutic actions will feature the oscillation frequencies that are applied to the patient's organism from outside.

To illustrate a relationship between the oscillation frequencies generated by the cells and the ratio between the amount of protein molecules differing in frequency that have been pulled towards the membrane, given below are some experimental findings. It is known commonly that EHF-radiations are capable of producing an effect at the cell level. That is why the experiments referred to hereinbelow have been made on cell suspensions, the regularities observed holding true of multicellular organisms as well. The culture cells of Saccharomyces carlsbergensis yeast were exposed to a 30-minute effect of electromagnetic oscillations at a frequency of 42.3 GHz, and then to the effect of electromagnetic oscillations at a frequency of 53.5 GHz for another hour, whereupon the cells were found to generate oscillations at a frequency of 53.5 GHz.

Conversely, when the cells were first exposed to the effect of oscillations at a frequency of 42.3 GHz for an hour, and then for further 30 min to the effect of oscillations at a frequency of 53.5 GHz, the cells were found to generate oscillations at a frequency of 42.3 GHz.

In the case where the exposure at the frequencies of 42.3 GHz and 53.5 GHz was equal in time, the result was found to be unstable, so that the irradiated cells may generate any of the frequencies mentioned above after the radiation had been discontinued.

The method for EHF-therapy, according to the invention, consists in that a radiation signal is shaped as alternation of an effective EHF-radiation signal and a spacing, the duration of said effective signal being selected within 30 and 1200 s.

The limit values of the duration of irradiation in case of an intermittent time behaviour (between 30 s and 20 min) can be explained as follows.

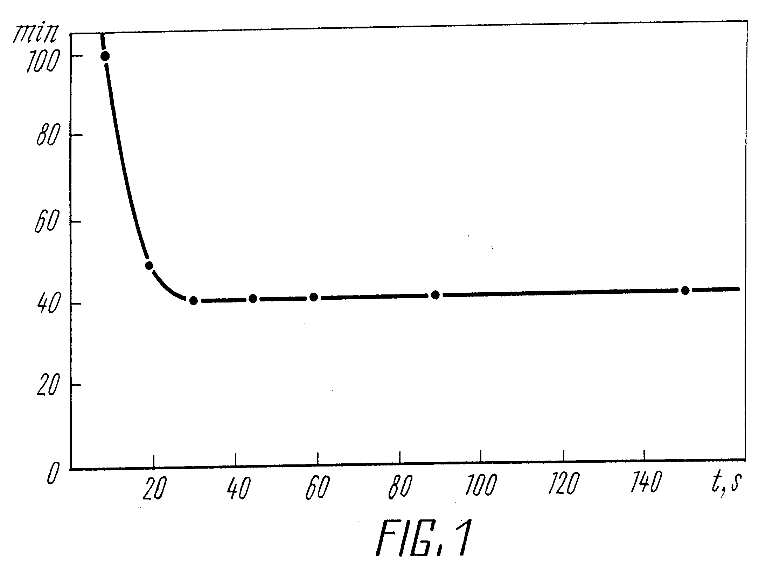

When an exposure time is shorter than 30 s the mode is not practically memorized by the cells. As a result, time required for attaining a biological effect with a shorter duration of exposures, starts increasing. All stated above is illustrated in FIG. 1, which represents the relationship between the time required for complete synchronization of a culture of the yeast mentioned above, and the duration of excitation with a constant duration of time spacing equal to 5 min , the density of an energy flux being 0.03 mW/cm$^2$, and the working frequency equalling 42.3 GHz.

Contrariwise, with the duration of irradiation exceeding 20 min any difference between the results obtained between the continuous and intermittent modes practically, disappears, since a characteristic total time required for attaining the desired biological effect is about 30 min.

Considered hereinbelow are exemplary embodiments of the proposed method, and their results are compared with those obtained from a continuous mode of irradiation. In the case of a continuous (uninterrupted) exposure to the effect of EHF-radiation complete synchronization was attained for 42 min. In a first mode an effective signal, at a frequency of 42.3 GHz and an energy flux density of 0.03 mW/cm$^2$, was repeated threefold, each signal lasting for 3 min, while two time spaces 15 min each were provided between said signals.

In a second and a third mode of EHF-action at the same frequency value and energy flux density, the signal was repeated fivefold, and sevenfold the duration of each being 2 min, while the signals were interrupted by a five-minute and a three-minute time intervals, respectively.

It ensues from the evidence given above that construction of substructures is not completed by the period of an external effect, but the process continues during time intervals between the signals. It is however essential that a total duration of the effective signal necessary for obtaining a definite biological effect is reduced by more than fourfold as compared with the continuous mode. Hence the number of protein molecules making part of protein substructures and having resonance frequencies corresponding to the oscillation frequencies of external effective signals, is reduced likewise nearly fourfold.

Table 1 contains data on two modes, which were used in the course of clinical trialling of the method, one of said modes comprising three cycles, each of which consisting of three effective signals lasting 3 min each and two 15-minute intervals, a second mode incorporated five cycles, each of which consisting of five 2-minute effective signals and four 5-minute intervals.

The biological criterion for assessment of the effect produced by the method, is complete synchronization of oscillations generated by the cells subjected to the effect of EHF-radiation.

As can be seen from Table 1, complete synchronization is attained by the proposed method for a total time of external EHF-radiation effect shorter than in the known method.

The proposed method for EHF-therapy is expedient to be applied in cases of extremely severe forms of the disease.

In cases of peptic ulcer of the stomach and duodenum (the majority of statistical data concerning continuous-mode EHF-therapy refers to this nosologic entity), patients with extremely severe forms of the diseases are assumed those with the duration of the disease exceeding 10 years and the recurrence incidence in excess of three times within a year, as well as the patients with complications of peptic ulcer, primarily such as bleeding and perforation. Complete healing of ulcers in such patients subjected to treatment with continuous-mode EHF-radiation requires as a rule several courses of treatment with EHF-radiation separated by two-or three-week intervals. The recommended treatment schedule is as follows: 10 to 15 sessions of EHF-therapy, two- or three-weak interval, a total number of treatment courses being not in excess of three. Patients with mild forms of the disease require approximately 10 sessions of EHF-treatment for complete healing of ulcers.

Table 2 given below contains data characteristic of the duration of the healing process in patients with extremely severe forms of peptic ulcer of the stomach and duodenum who were subjected to EHF-therapy in the proposed mode of radiation.

It is obvious from Table 2 that the treatment period with EHF-radiation of the critically ill patients becomes equal to the period of treatment of the slightly ill patients and those with moderate-severity form of the disease when using a continuous mode of EHF-radiation.

Similar results have been obtained when the proposed method is applied for treatment of some other disease entities. For instance, good and very good results have been obtained in 75 percent of cases when EHF-therapy is applied for treatment of angina pectoris by a continuous-mode of EHF-radiation in patients of the first and second functional classes of disease severity. However, the percent of good results of treatment of the patients of the fourth functional class of the disease is reduced to 10.

The results of treatment of the patients of the fourth functional class of disease severity approximate those of the patients belonging to the first and second functional classes of disease severity when a continuous mode of radiation is employed.

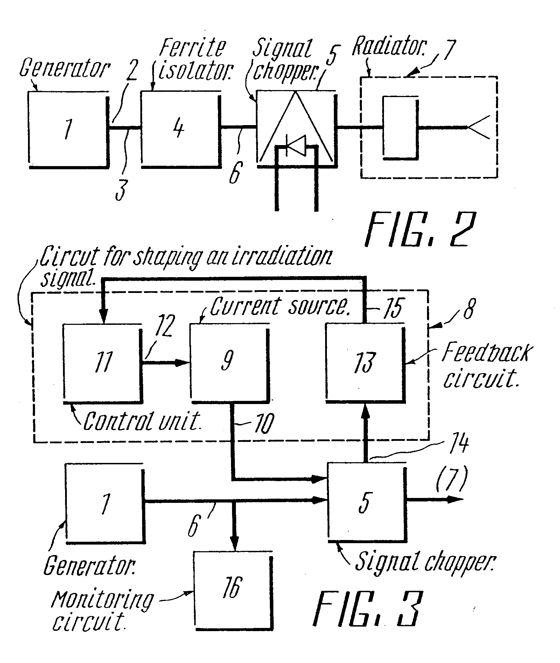

FIG. 2 presents a block diagram of the device for carrying the proposed method into effect.

The device comprises a generator 1 of nonthermal intensity EHF-radiation, whose output 2 is connected, through a portion 3 of the EHF-channel, to a ferrite isolator 4. The device incorporates also a signal chopper 5, whose input is connected, via a portion 6 of the EHF-channel, to the output of the isolator 4. The pulse chopper 5 is in fact a diode modulator based on a p-i-n diode and is connected, through the portion 6 of the EHF-channel, to a radiator means hereinafter referred to as a radiator 7, which appears as a horn antenna.

FIG. 3 shows a block diagram of a means 8 for shaping an irradiation signal in the form of alternating an effective signal and a spacing interval, wherein duration of each effective signal is less than or equal to the duration of the spacing interval that follows said effective signal. The means 8 is interconnected with the pulse chopper 5 and comprises a current source 9, which is connected to the chopper 5 through its output 10, a unit 11 for control of the current source 9, said unit being connected, through its output 12, to the input of the current source 9, and a feedback circuit 13 connected, through its input, to an electric output 14 of the chopper 5 and via its output 15, to the control unit 11.

The device comprises also an EHF-radiation signal monitoring circuit 16, which is connected to the portion 6 of the EHF-channel.

The device for EHF-therapy of human disease functions as follows. EHF-radiation produced by the generator 1 is fed along the portion 3 of the EHF-channel to the isolator 4, and from its output the radiation is passed along the portion 6 of the EHF-channel to the chopper 5.

Then a signal shaped by the means 8 is applied to the chopper 5. To this end the control unit 11 shapes a signal for turning in or out the current source 9, from which a command is sent for the chopper 5 to operate. At the same time the state of the chopper 5 is monitored by the feedback circuit 13, which delivers a signal from its output 15 that is in fact a monitoring signal indicating that the command is executed.

Next a signal of EHF-radiation shaped at the output of the chopper 5, arrives at the radiator 7, from whence it is applied to the patient's organism.

The EHF-radiation signal appearing at the output of the chopper 5 is in effect an alternation of the effective EHF-radiation signal and a spacing interval.

The proposed method and device are instrumental in attaining higher efficiency of treatment of patients, especially when inveterate diseases or severe affections of patient's organism are to be treated, as well as in reducing the treatment period.

Table 1

| Mode | Parameters | Total irradiation time | Oscillation frequency | Irradiation | Amount of synchronization |
|------|------------|------------------------|-----------------------|-------------|---------------------------|
| 1 | 2 | 3 | 4 | 5 | 6 |
| | Three cycles | | | | |
| | 3 min- irradiation | 9 min | 42.3 GHz | 0.03 mW/cm$^2$ | Complete synchronization |
| I | 15 min- interval | | | | at frequency |
| | 3 min – irradiation | | | | of 42.3 GHz |
| | 15 min – interval | | | | |
| | 3 min – irradiation | | | | |
| | Five cycles | 10 min | Same | Same | Same |
| | 2 min – irradiation | | | | |
| | 5 min – interval | | | | |
| | 2 min – irradiation | | | | |
| | 5 min – interval | | | | |
| II | 2 min – irradiation | | | | |
| | 5 min – interval | | | | |
| | 2 min – irradiation | | | | |
| | 5 min – interval | | | | |
| | 2 min – irradiation | | | | |

Table 1 (continued)

| 1 | 2 | 3 | 4 | 5 | 6 |
|---|---|---|---|---|---|
| | Seven cycles | 14 min | Same | Same | Same |
| | 2 min - irradiation | | | | |
| | 3 min - interval | | | | |
| | 2 min - irradiation | | | | |
| | 3 min - interval | | | | |
| III | 2 min - irradiation | | | | |
| | 3 min - interval | | | | |
| | 2 min - irradiation | | | | |
| | 3 min - interval | | | | |
| | 2 min - irradiation | | | | |
| | 3 min - interval | | | | |
| | 2 min - irradiation | | | | |
| | 3 min - interval | | | | |
| | 2 min - irradiation | | | | |
| IV Continuous | Same | 42 min | Same | Same | Same |

Table 2

| Pati-ent's conven-tional No | Ulcer locali-zation | Ulcer size, cm | Number of re-lapses per year | Aggrava-ting fact |
|---|---|---|---|---|
| 1 | 2 | 3 | 4 | 5 |
| 1 | Duodenal bulb | 0.6 | 4 | – |
| 2 | Duodenal bulb | 1.2 | 2 | Bleeding |
| 3 | Stomach | 3.0 | 2 | Bleeding |
| 4 | Stomach | 1.5 | 2–3 | – |
| 5 | Duodenal bulb | 0.5 | 3 | – |
| 6 | Duodenal bulb | 0.8 | 2–3 | – |
| 7 | Duodenal bulb | 0.5 | 3 | – |
| 8 | Duodenal bulb | 0.8 | 2–3 | – |
| 9 | Duodenal bulb | 0.3 and 0.5 | 2 | Multiple ulcers |
| 10 | Duodenal bulb | 0.8 | 2 | Bleeding |
| 11 | Duodenal bulb | 0.5 | 2 | Perfora-tion |
| 12 | Duodenal bulb | 0.5 | 3 | – |

Table 2 (continued)

| No | Duration of disease, years | Age, years | Treatment results, num-ber of sessions |
|---|---|---|---|
| 1 | 6 | 7 | 8 |
| 1 | 32 | 67 | Healing after 20 sessions |
| 2 | 9 | 48 | Healing after 20 sessions |
| 3 | 11 | 47 | Healing after 25 sessions |
| 4 | 13 | 51 | Healing after 10 sessions |
| 5 | 14 | 26 | Healing after 10 sessions |
| 6 | 17 | 38 | Healing after 10 sessions |
| 7 | 22 | 39 | Healing after 20 sessions |
| 8 | 17 | 52 | Healing after 17 sessions |
| 9 | 7 | 44 | Healing after 15 sessions |
| 10 | 26 | 48 | Healing after 17 sessions |
| 11 | 6 | 43 | Healing after 10 sessions |
| 12 | 25 | 45 | Healing after 10 sessions |

LIST OF REFERENCE NUMERALS USED IN THE DRAWINGS

1 - generator of EHF-radiation

2 - output of the generator 1

3 -     portion of the EHF-channel
4 -     ferrite isolator
5 -     signal chopper
6 -     portion of the EHF-channel
7 -     radiator
8 -     circuit for shaping an irradiation signal
9 -     current source
10 -    output of the source 9
11 -    control unit
12 -    output of the control unit 11
13 -    feedback circuit
14 -    output of the chopper 5
15 -    output of the feedback circuit 13
16 -    monitoring circuit

## Claims

1.  A method for EHF-therapy of human diseases, consisting in that there is selected nonthermal-intensity EHF-radiation, a radiation signal is shaped in the form of alternating an effective EHF-radiation signal and a spacing, wherein the duration of each effective signal is shorter than the duration of said spacing that follows said effective signal, and a patient's organism is exposed to the effect of the thus-shaped radiation signal so as to obtain a curative effect.

2.  A method as claimed in Claim 1, wherein the duration of said effective signal is selected from 30 to 1200 s.

3.  A device for EHF-therapy of human diseases, comprising:
    -   a nonthermal-intensity EHF-radiation generator having an output;
    -   a first portion of an EHF-channel connected to said output of said generator and transmitting EHF-radiation from said generator;
    -   a signal chopper, having an input and an output and connected to said first portion of the EHF-channel through said input;
    -   a second portion of the EHF-channel connected to said output of said signal chopper;
    -   a means for shaping an irradiation signal appearing as an alternation of an effective EHF-signal and a spacing, wherein the duration of each effective signal is shorter than the duration of the spacing that follows said effective signal, said means being connected to said signal chopper; and
    -   a radiator means connected to said second portion of the EHF-channel and applying an irradiation signal to the patient's organism.

FIG. 1

EP 0 563 428 A1

FIG. 2

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 293 080 (TAKASE) <br> * abstract * <br> --- | 1,3 | A61N5/02 |
| A | US-A-4 741 348 (KIKUCHI) <br> * column 7, line 66 - column 8, line 33 * <br> ----- | 1,3 | |
| | | | TECHNICAL FIELDS SEARCHED (Int. Cl.5) |
| | | | A61N |

The present search report has been drawn up for all claims

| Place of search | Date of completion of the search | Examiner |
|---|---|---|
| THE HAGUE | 18 NOVEMBER 1992 | TACCOEN J-F.P.L. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)